# EUROPEAN PATENT APPLICATION

(11) **EP 2 025 343 A1**
(43) Date of publication of application: **18.02.2009**
(21) Application number: 08162311.8
(22) Date of filing: 13.08.2008
(51) Int. Cl.: A61K 38/17, A61P 35/00

(54) **A short-chain peptide anti-cancer drug and its use**

(30) Priority: 14.08.2007 TW 96129940
(71) Applicant: Asia-Pacific Biotech Developing Inc., KaoHsiung (TW)
(72) Inventor: Chang, Fu-Hsin, KaoHsiung (TW)
(74) Representative: Viering, Jentschura & Partner

(57) **Abstract**

The present invention relates to a short-chain peptide anti-cancer drug and its use to maintain the stability and safety of cancer therapy. The short-chain peptide is extracted from a protein associated with mitosis and has molecular weight less than 60KDa such that the short-chain peptide cannot permeate a normal blood vessel that has a smaller porous size and will not damage normal cells but will be delivered and diffused to cancer cells by an anti-angiogenesis treatment to inhibit cancer cell growth.

## Description

### FIELD OF THE INVENTION

The invention relates to a short-chain peptide cancer drug and its use, particularly to use a short-chain peptide that quickly diffuses and accumulates in a new blood vessel of a cancer cells to kill cancer cells.

### BACKGROUND OF THE INVENTION

Three conventional therapies exist to treat cancer cells, surgery, radiotherapy and chemotherapy. Surgery is used to remove cancer cells in early stages of the disease. Before performing surgery, a patient's age and physical conditions should be taken into consideration since different side effects will be induced. Radiotherapy is also called palliative radiotherapy and is used to kill cancer cells in an isolated region through an irradiation but will also injury other cells in the region. Even though radiotherapy is therapeutically effective, huge side effects exist with the therapy. Chemotherapy is always used to treat patients that have undergone surgery to remove most cancer cells or have had cancer cells metastasized through injection of chemical drugs (anti-cancer drugs). Those chemical drugs will kill both normal cells and cancer cells. Patients with some renal insufficiency are often restricted to chemotherapy. Most patients undergoing chemotherapy will be nauseous, lose hair or generally feel uncomfortable.

Because biotechnology has developed so rapidly, scientists have been researching more therapeutic cancer treatments. Gene therapy is a promising and popular field. Gene placement is a gene therapy technique and uses a recombinant gene to replace damaged genes that cause genetic diseases. Efficiency is an important issue in gene therapy, such as developing a carrier with high stability to deliver the recombination gene to a targeted cell to replace damaged genes. Furthermore, safety of inserting the recombination gene in the targeted cell is another issue. A shortcoming of gene therapy is that replacement of a gene may change the original genetic expression, so gene therapy is used only in somatic cells, not in germ cells.

When cancer cells exceed 2mm in diameter, the cancer cells or connective tissue around the cancer cells will secret a compound to stimulate angiogenesis that is the fast growth of new blood vessels from epidermal cells of pre-existing vessels so the cancer cells can obtain nutrients. Therefore, the new blood vessels will affect the growth and metastasize of cancer cells. An anti-angiogenesis treatment uses anti-cancer drugs to interrupt nutrient delivery to cancer cells with high permeable new blood vessels having pore size of 60nm to 100 Å. The new blood vessels has bigger pore size that permit the anti-cancer drug to diffuse into the cancer cells to prevent angiogenesis and metastasis. Identifying and localizing specific cancer cells before injecting anti-cancer drugs would be better. The anti-angiogenesis treatment should be used on specific tissue and be isolated from surrounding tissue because it also affects the angiogenesis of normal cells. However, efficiency of the anti-angiogenesis treatment has not been firmly established.

The inventors have created the present invention to prevent the disadvantages of surgery, chemotherapy and radiotherapy and to improve the stability and safety of gene therapy and anti-angiogenesis treatment.

### SUMMARY OF THE INVENTION

The objective of the present invention relates to a short-chain peptide anti-cancer drug and its use to maintain the stability and safety of cancer therapy.

The short-chain peptide in accordance with the present invention is extracted from a protein associated with mitosis and has a molecular weight less than 60KDa such that the short-chain peptide cannot permeate a normal blood vessel that has a smaller pore size and will not damage normal cells but will be delivered and diffused to cancer cells by an anti-angiogenesis treatment to inhibit cancer cell growth.

### BRIEF DESCRIPTION OF THE DRAWINGS

Fig. 1 is a graph of the survival rate of the cancer cell when a short-chain peptide anti-cancer drug in accordance with the present invention has not been used.
Fig. 2 is a graph of the survival rate of the cancer cell after the short-chain peptide anti-cancer drug has been added.
Fig. 3(a) is a graph of the life cycle of the cancer cells before the short-chain peptide anti-cancer drug has been added.
Fig. 3(b) is a graph of the life cycle of the cancer cells after the short-chain peptide anti-cancer drug has been added.
Fig. 3(c) is a graph of the life cycle of the cancer cells 24 hours after adding the short-chain peptide anti-cancer drug.

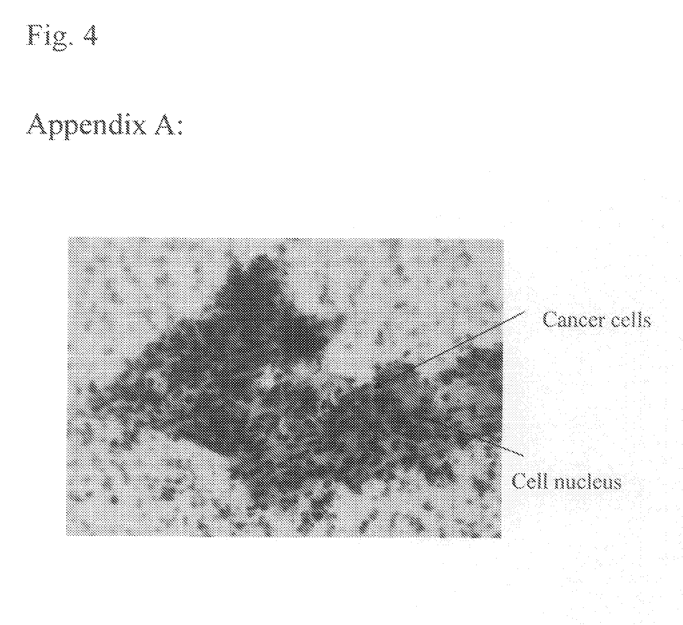

Fig. 4 is a graph of the cancer cells status before adding the short-chain peptide anti-cancer drug.

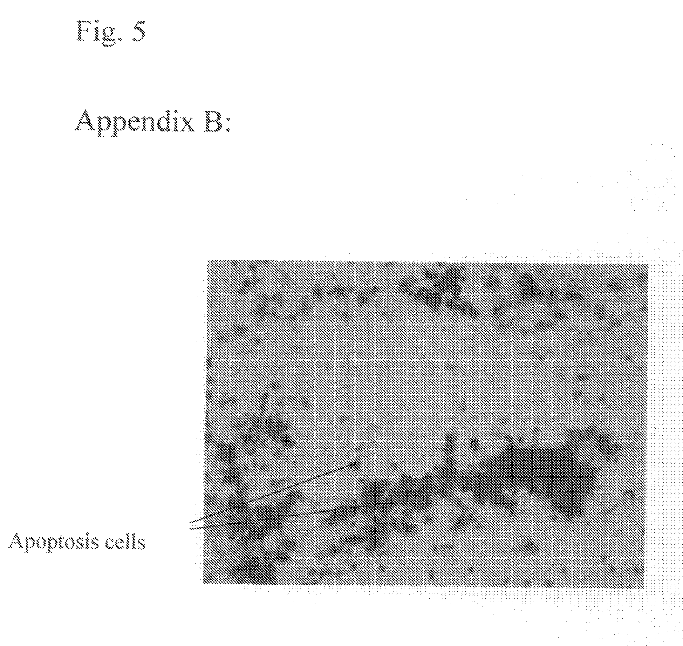

Fig. 5 is a graph of the cancer cells status after adding the short-chain peptide anti-cancer drug.

### DETAILED DESCRIPTION OF THE PRESENT INVENTION

The present invention is a short-chain peptide anti-cancer drug. A short-chain peptide in accordance with the present invention is extracted from a protein associated with mitosis and has a molecular weight less than 60KDa such that the short-chain peptide cannot permeate a normal blood vessel that has a smaller pore size and will not damage healthy cells but will be delivered and diffused to cancer cells by an anti-angiogenesis treatment to inhibit cancer cell growth.

The short-chain peptide of present invention may consist of 48 amino acids and comprises a sequence of:
Arginine-Glycine-Aspartic acid-Leucine-Alanine-Serine-Leucine-Glutamine-Lysine-Alanine-Alanine-Histidine-Glycine-Histidine-Valine-Argini ne-Lysine-Alanine-Phenylalanine-Lysine-Serine-Histidine-Valine-Serine-Thre onine-Leucine-Threonine-Aspartic acid-Leucine-Glutamine-Proline-Tyrosine-Methionine-Arginine-Glutamine-Phenylalanine-Valine-Alanine-Histidine-Leuc ine-Glutamine-Glutamic acid-Threonine-Serine-Proline-Leucine-Arginine-Aspartic acid (also described as R-G-D-L-A-S-L-Q-K-A-A-H-G-H-V-R-K-A-F-K-S-H-V-S-T-L-T-D-L-Q-P-Y-M-R-Q-F-V-A-H-L-Q-E-T-S-P-L-R-D).

The short-chain peptide is extracted from a protein associated with mitosis, is modified and can inhibit cancer cells from producing the protein for cancer cell mitosis. Cancer cells' growth will stop at the mitosis phage without the protein and will induce apoptosis that will cause cancer cell death.

A short-chain peptide anti-cancer drug treatment in accordance with the present invention comprises
(A) injecting a short-chain peptide anti-cancer drug;
(B) the short-chain peptide anti-cancer drug diffusing and accumulating at new blood vessels of cancer cells; and
(C) the short-chain peptide anti-cancer drug inhibiting the cancer cell growth.

The treatment uses the short-chain peptide anti-cancer drug through an anti-cancer angiogenesis targeted treatment that uses permeability of the blood vessel of a normal cells and a cancer cell to control delivery of the short-chain peptide anti-cancer drug to cancer cells. The short-chain peptide anti-cancer drug's particle size is smaller than pores of a new blood vessel of a cancer cell and larger than pores of a blood vessel of a normal cell. The short-chain peptide anti-cancer drug will enter and accumulate at new blood vessels of cancer cells. The short-chain peptide can inhibit cancer cells' apoptosis and kill the cancer cells.

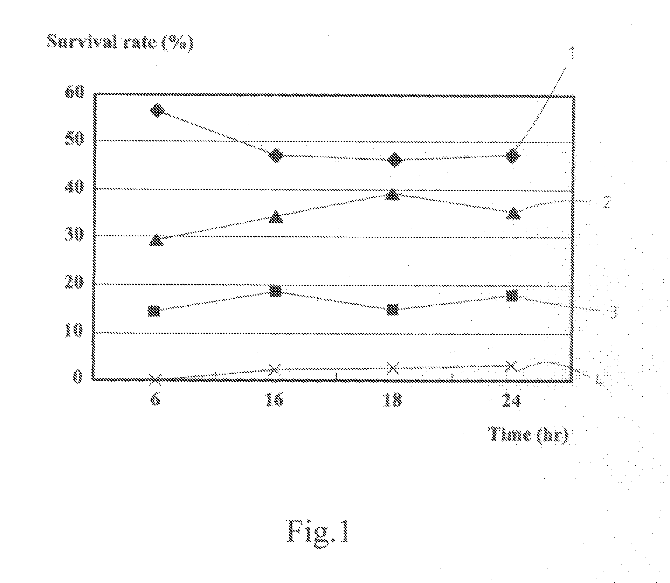

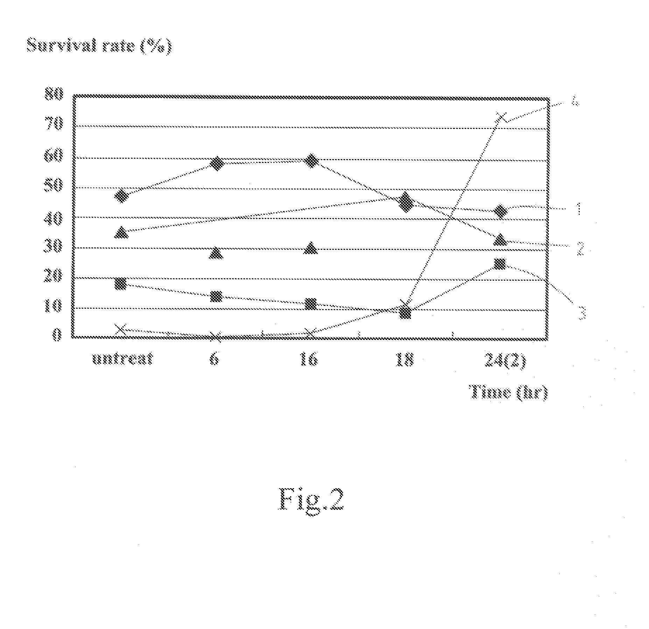

With reference to Figs. 1 and 2, without adding, the survival rate of cancer cells when the short-chain peptide anti-cancer drug has not been added is about 95%. 24 hours after adding the short-chain peptide anti-cancer drug, the cancer cells start to apoptosis, and the survival rate of cancer cells is about 25%. Line 1, 2, 3 or 4 indicates the survival rate of tumor cells in S, diploid G1, diploid G2 or death phase of mitosis respectively.

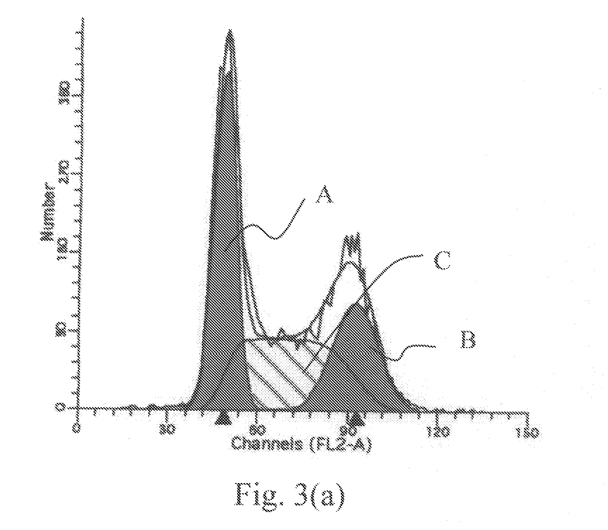

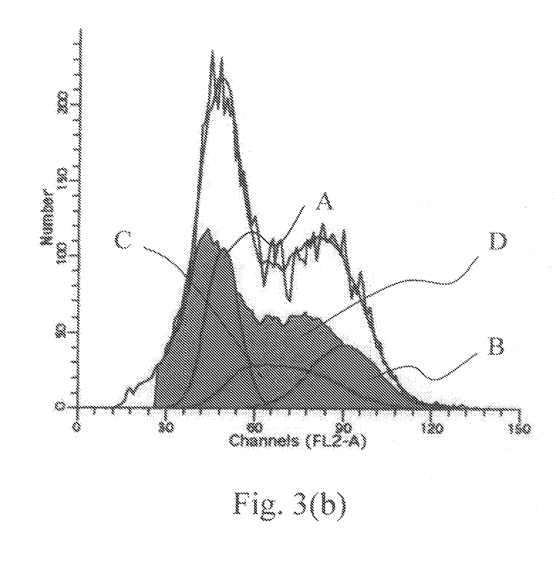

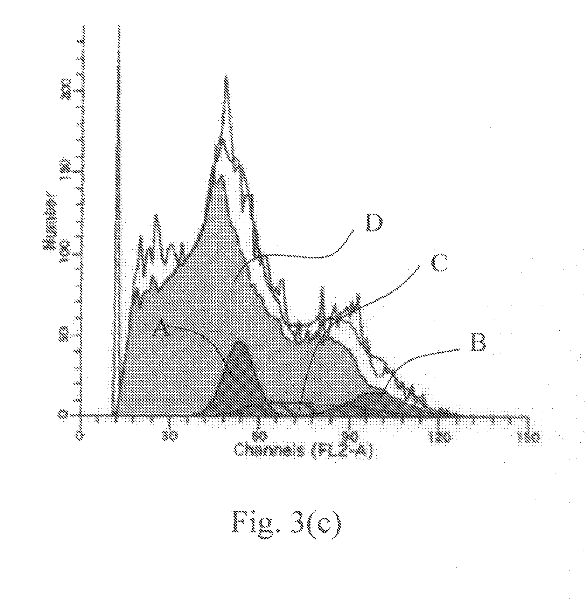

With reference to Figs. 3(a) to 3(c), the short-chain peptide anti-cancer drug indeed inhibits cancer cells growth and after 24 hours incubation, with about 70% cancer cell death. Area A, B, C and D indicates the number of cells in G1, G2, S and death phase of mitosis. The short-chain peptide anti-cancer drug can induce cancer cells apoptosis. The appendix A and B show the microscopic graph of the cells.

## Claims

1. A short-chain peptide anti-cancer drug extracted from a protein associated with mitosis comprising a short-chain peptide has molecular weight less than 60KDa.

2. The anti-cancer drug as claimed as claim 1, wherein the short-chain peptide comprises a sequence of
Arginine-Glycine-Aspartic acid-Leucine-Alanine-Serine-Leucine-Glutamine-Lysine-Alanine-Alanine-Histidine-Glycine-Histidine-Valine-Argini ne-Lysine-Alanine-Phenylalanine-Lysine-Serine-Histidine-Valine-Serine-Thre onine-Leucine-Threonine-Aspartic acid-Leucine-Glutamine-Proline-Tyrosine-Methionine-Arginine-Glutamine-Phenylalanine-Valine-Alanine-Histidine-Leuc ine-Glutamine-Glutamic acid-Threonine-Serine-Proline-Leucine-Arginine-Aspartic acid.

3. The anti-cancer drug as claimed as claim 1, wherein the anti-cancer drug is delivered by an anti-cancer angiogenesis targeted treatment.

4. A short-chain peptide anti-cancer drug treatment comprising:
(A) injecting a short-chain peptide anti-cancer drug;
(B) the short-chain peptide anti-cancer drug diffusing and accumulating in new blood vessels of a cancer cell; and
(C) the short-chain peptide anti-cancer drug inhibiting the cancer cell growth.

5. The method as claimed as claim 4, wherein the short-chain peptide comprises a sequence of:
Arginine-Glycine-Aspartic acid-Leucine-Alanine-Serine-Leucine-Glutamine-Lysine-Alanine-Alanine-Histidine-Glycine-Histidine-Valine-Argini ne-Lysine-Alanine-Phenylalanine-Lysine-Serine-Histidine-Valine-Serine-Thre onine-Leucine-Threonine-Aspartic acid-Leucine-Glutamine-Proline-Tyrosine-Methionine-Arginine-Glutamine-Phenylalanine-Valine-Alanine-Histidine-Leuc ine-Glutamine-Glutamic acid-Threonine-Serine-Proline-Leucine-Arginine-Aspartic acid.

6. The method as claimed as claim 4, wherein the anti-cancer drug is delivered by an anti-cancer angiogenesis targeted treatment.
